# EUROPEAN PATENT SPECIFICATION

(11) **EP 2 836 207 B1**
(45) Date of publication and mention of the grant of the patent: **19.02.2020**
(21) Application number: 13775814.0
(22) Date of filing: 12.04.2013
(51) Int. Cl.: A61K 9/48, A61K 31/496, A61K 31/47, A61K 31/137, A61K 31/495, A61K 31/4178, A61K 31/4422, A61K 31/505, A61K 31/616, A61K 31/4365

(54) **COMPOSITE FORMULATION COMPRISING MULTI-UNIT SPHEROIDAL TABLET (MUST) ENCAPSULATED IN HARD CAPSULE AND METHOD FOR PREPARING SAME**
ZUSAMMENGESETZTE FORMULIERUNG MIT KUGELFÖRMIGEN TABLETTEN AUS MEHREREN EINHEITEN IN EINER HARTKAPSEL UND VERFAHREN ZUR HERSTELLUNG DAVON
FORMULATION COMPOSITE COMPRENANT DES COMPRIMÉS SPHÉROÏDAUX À PLUSIEURS UNITÉS (MUST) ENCAPSULÉS DANS UNE CAPSULE DURE ET SON PROCÉDÉ DE PRÉPARATION.

(30) Priority: 13.04.2012 KR 20120038594
(43) Date of publication of application: 18.02.2015
(73) Proprietor: Hanmi Pharm. Co., Ltd., Gyeonggi-do 445-910 (KR)
(72) Inventor: KIM, Kyeong Soo, Suwon-si Gyeonggi-do 440-710 (KR); KIM, Dong Ho, Seongnam-si Gyeonggi-do 463-853 (KR); KWON, Taek Kwan, Suwon-si Gyeonggi-do 440-825 (KR); KIM, Yong Il, Suwon-si Gyeonggi-do 442-872 (KR); PARK, Jae Hyun, Suwon-si Gyeonggi-do 443-737 (KR); WOO, Jong Soo, Suwon-si Gyeonggi-do 440-330 (KR)
(74) Representative: Marks & Clerk (Luxembourg) LLP
(86) International application number: PCT/KR2013/003099
(87) International publication number: WO 2013/154390

(56) References cited:
- EP-A1- 1 462 098
- WO-A1-97/25064
- WO-A1-2012/011882
- WO-A2-03/103637
- WO-A2-2009/118359
- WO-A2-2012/124973
- KR-A- 20070 072 982
- KR-A- 20080 032 616
- KR-A- 20090 091 076
- KR-A- 20090 091 080
- "A PHARMACEUTICAL COMPOSITION COMPRISING MONTELUKAST SODIUM AND LEVOCETIRIZINE DIHYDROCHLORIDE IN A SINGLE DOSAGE FORM", IP.COM JOURNAL, IP.COM INC., WEST HENRIETTA, NY, US, 15 May 2008 (2008-05-15), XP013124986, ISSN: 1533-0001
- LEELA KEERTHI ET AL: INTERNATIONAL JOURNAL OF PHARMACEUTICAL SCIENCES REVIEW AND RESEARCH PAGE, vol. 28, no. 1, 1 September 2014 (2014-09-01), pages 214-221, XP055201354, ISSN: 0976-044X

## Description

### FIELD OF THE INVENTION

The present invention relates to a hard capsule composite formulation comprising multi-unit spheroidal tablets (MUSTs) and method for preparing same.

### BACKGROUND OF THE INVENTION

The advancement in the field of medicine has improved the quality of life and increased the life expectancy of humans. However, there is a limit to the efficacy of a single pharmaceutically active ingredient in treating patients with medical disorders. Thus, it is common to administer multiple medications having different mechanisms (modes) of action simultaneously or sequentially for synergistic effects.

However, co-administration of two or more separate drug units may reduce patents' compliance to taking medicine, thereby causing great inconvenience to the patients who are subjected to continuous drug treatments. Further, the patients have to take such multiple drug units at once, and carry them all the time. It will also give the patients great inconvenience in their daily lives.

In order to redress such problems, there has been suggested a method of packaging a number of medications in a single package. For instance, Torrent Pharmaceuticals Ltd. (India) has released a composite formulation "CVpill," a single kit containing a capsule and a tablet for treating a cardiovascular disease. CVpill consists of a capsule containing 10 mg of Atorvastatin in powder form, Ramipril in powder form and 75 mg of an enteric-coated aspirin tablet, and a sustained-release tablet containing 50 mg of Metoprolol. The capsule and the tablet must be administered once a day simultaneously. But such co-packaged products consisting of a simple kit can hardly improve patients' compliance to taking medicine, which, however, may be expected in a composite formulation. Therefore, there is a growing demand for a research on development of a "combination drug or composite formulation" of specific active ingredients.

WO 2012/124973 discloses a combined formulation for oral administration for treatment of cardiovascular disease, comprising (a) cholesterol lowering agent mini-tablets having a diameter of 7.5 mm or less, which contain cholesterol lowering agent, a stabilizer thereof and a pharmaceutically acceptable excipient and have a coating layer on a surface thereof, and (b) antithrombic agent mini-tablets or mini-pellets having a diameter of 7.5 mm or less, which contain an antithrombotic agent a pharmaceutically acceptable excipient thereof and include an enteric coating film on a surface thereof.

EP 1 462 098 is related to a solid pharmaceutical composition comprising two separate regions, a first region comprising at least one non-steriodal anti-inflammatory drug (NSAID) and an adequate pharmaceutical carrier containing a retardant material for an extended release delivery of said non-steriodal anti-inflammatory drug (NSAID), and a second region comprising a stabilized gastroprotective prostaglandin and an adequate pharmaceutical carrier containing a retardant material for an immediate release of said stabilized gastroprotective prostaglandin.

The IP.com Jounral, IPCOM000170324D, 2008 relates to a pharmaceutical composition comprising montelukast sodium and levocetririzine dihydrochloride in a single dosage form, designed to minimize interaction between the two drugs.

The term "composite formulation" as used herein, refers to a combination of two or more different active ingredients or drugs in a single unit dose such as tablet or capsule. However, development of a composite formulation for specific active ingredients is sometimes very difficult for the following reasons.

First, the combination of specific active ingredients to be used for a composite formulation should readily be made. Further, the composition comprising active ingredients and a pharmaceutically acceptable excipient should be in appropriate size and weight for administration thereof. However, it is not always easy to develop a composite formulation which meets such requirements. If the amount of drugs to be employed is excessive or insufficient, it would be difficult to adjust the weight of the composition to an appropriate level. Also, unexpected problems may be encountered in the course of dealing with the various conditions resulting from the pharmacokinetical and pharmaceutical properties of drugs.

Second, the chemical interaction between the active ingredients in the preparation of a composite formulation may reduce the stability of drugs. Especially, it is even more difficult to develop a fixed combination dosage form with sufficient physicochemical stability for a combination of drugs if the stability thereof may be reduced due to their chemical interaction when combined.

When a composite formulation of tablet is prepared, a double- or triple-layer tableting machine can be used to separate the active ingredients. Moreover, not only does such method require special equipments, but it is also mechanically impossible to completely separate the main ingredients in each layer since an undesirable reaction may take place at the interface of the layers.

For a capsule, a conventional hard capsule is charged with drugs in the form of powder, granule or pellet. Also, only a single active ingredient is charged in a hard capsule by a single charging step. Also, a drug in the form of powder, granule or pellet has a density lower than that of a tablet since the former is not subjected to a high pressure compressing step. Thus, there exists a limit in the amount of drugs in the form of powder, granule or pellet to be charged in a capsule. In order to charge a high dose of an active ingredient or more than one active ingredient in a single hard capsule, the size of capsule must be increased to accommodate such large amount of drugs. However, if the size of the capsule becomes too big for accommodating a large amount of drugs, it may cause swallowing difficulties, dysphagia. Particularly, capsules having large sizes of No. 00 (8.5 mm in capsule cap diameter and 23.3 mm in capsule length) and No. 0 (7.6 mm in capsule cap diameter and 21.7 mm in capsule length) may cause difficulties for elderly people or children to swallow them. It may also be inconvenient to carry them due to their large size.

Accordingly, the present inventors have endeavored to resolve the disadvantages of a composite formulation and have developed a hard capsule comprising a small number of, e.g., 1 to 3, tablets per main ingredient. However, the initial dissolution rate (within 15 min) of the hard capsule slowed down due to a delay in the disintegration time of the hard capsule, and an increase in the deviation between individual dissolution test results was observed. Thus, it may be difficult to expect the same bioequivalence of a single dosage form from a composite formulation if it comprises a drug which requires fast absorption rate, e.g., maximum drug concentration time (Tₘₐₓ) of 1 to 2 hrs. Therefore, there still remains a need for developing a composite formulation with good productivity and stability which does not have a delay in initial dissolution rate.

### SUMMARY OF THE INVENTION

Therefore, it is an object of the present invention to provide a composite formulation having no delay in initial dissolution rate within 15 min, and exhibiting good *in vivo* absorption rate owing to a small change in each dissolution rate, as well as good productivity and stability.

It is another object of the present invention to provide a method for preparing the composite formulation.

In accordance with one object of the present invention, there is provided a hard capsule composite formulation according to claim 1.

In accordance with another object of the present invention, there is provided a method for preparing the hard capsule composite formulation according to claim 10.

The hard capsule composite formulation according to the present invention comprising multi-unit spheroidal tablets (MUSTs) can effectively charge the MUSTs in the limited space of the capsule, which allows charging a high dose of different pharmaceutically active ingredients in a capsule with a relatively small size, to thereby increase the productivity and render it readily administered to patients. The capsule has a good dissolution rate because the pharmaceutically active ingredients contained in the capsule are separated from one another; therefore, the dissolution rates of the ingredients are less affected by one another. It may also be possible to maximize the therapeutic effects of the pharmaceutically active ingredients since the composite formulation has good stability.

### BRIEF DESCRIPTION OF THE DRAWINGS

Fig. 1 shows a schematic view of a hard capsule composite formulation in accordance with one embodiment of the present invention.
Fig. 2 shows a schematic view of a multi-unit spheroidal tablet (MUST), which is charged in the hard capsule composite formulation.
Figs. 3 and 4 are graphs showing the dissolution rates of montelukast and levocetirizine, respectively, in accordance with Test Example 1.
Fig. 5 is a graph showing the dissolution rate of ambroxol in accordance with Test Example 2.
Figs. 6 and 7 are graphs showing the plasma levels of montelukast and levocetirizine, respectively, in accordance with Test Example 3.

### DETAILED DESCRIPTION OF THE INVENTION

Embodiments of the present invention are explained in detail hereinafter.

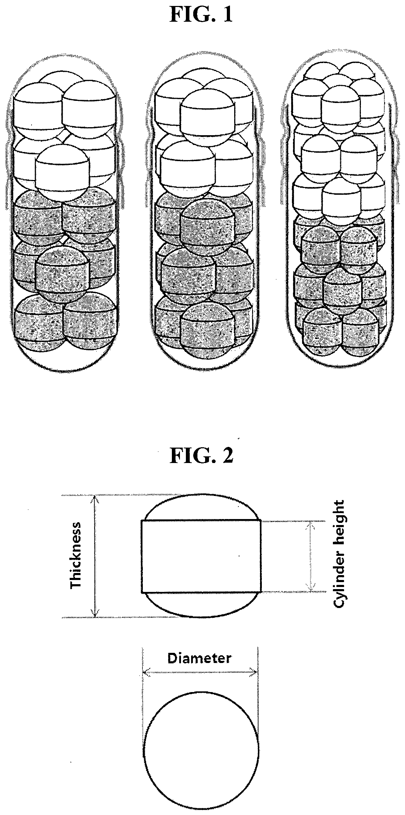

The present invention provides a hard capsule composite formulation comprising multi-unit spheroidal tablets (MUSTs). The schematic view of the hard capsule composite formulation in accordance with the present invention is shown in Fig. 1.

The term "composite formulation," as used herein, refers to a combination of two or more different active ingredients or drugs in a single unit dose. The composite formulation of the present invention comprises a capsule, and a plurality of multi-unit mini tablets, which contains any one of the pharmaceutically active ingredients, having a sphere-like shape that is encapsulated in the capsule.

In the inventive composite formulation, the capsule may be any conventional capsule used in the pharmaceutical industry, preferably a hard capsule. A hard capsule is composed of a capsule body and a cap, so that contents are charged in the internal space of the capsule body and the capsule cap is used to close the capsule body for use. The final capsule product, with its cap closed, that is actually administered to patients has the shape of cylindrical body with hemispherical ends, and the contents are charged in the internal space of the capsule body. In general, the internal space of conventional capsules is often charged with powder, granule or pellet in general, however, the internal space of the inventive composite formulation is characterized in being charged with mini tablets instead. The size of the mini tablets are small, and thus a plurality of mini tablets, e.g., 4 or more, may be charged in the internal space of the inventive composite formulation.

Capsules with various size numbers are used depending on the capsule size, but capsules having large size such as No. 00 (8.5 mm in capsule cap diameter and 23.3 mm in capsule length) may cause difficulties for elderly people or children to swallow them. It may also not portable due to their large size.

Therefore, the capsule size of the inventive composite formulation is preferably hard capsule No. 0 or smaller, e.g., hard capsule No. 0 (7.6 mm in capsule cap diameter and 21.7 mm in capsule length), hard capsule No. 1 (6.9 mm in capsule cap diameter and 19.1 mm in capsule length), hard capsule No. 2 (6.4 mm in capsule cap diameter and 17.6 mm in capsule length), hard capsule No. 3 (5.8 mm in capsule cap diameter and 15.7 mm in capsule length) or hard capsule No. 4 (5.3 mm in capsule cap diameter and 14.2 mm in capsule length), and more preferably the capsule size of hard capsule No. 1 or smaller.

In the inventive composite formulation, the MUST may comprise pharmaceutically active ingredients and pharmaceutically acceptable additives.

The MUST can be prepared by subjecting a mixture or granules of pharmaceutically active ingredients and pharmaceutically acceptable additives to a compression step with a tableting machine. In such case, the hardness of the tablet is determined by the magnitude of the compression pressure.

The tablet can be prepared in the form of a circle, rectangle or oval, however, a circular form is preferred because it allows easier packing in the internal space of the hard capsule. Particularly, when the diameter of the circular tablet is similar to the thickness of the circular tablet, it has a spheroidal shape, which improves the flowability of the tablet, and also minimizes the void to be formed in the capsule by forming a desirable packing arrangement.

Therefore, the tablet to be charged in the capsule is preferably in the form of a circle, more preferably a mini spheroidal tablet (MUST). The multi-unit spheroidal tablet which is comprised in the inventive composite formulation is shown in Fig. 2.

In order to prepare the circular tablet in the form of a spheroid, the ratio of the diameter of the circular tablet to the thickness thereof of each MUST needs to be in a range of 1 : 0.7 to 1 : 1.3, preferably 1 : 0.8 to 1 : 1.2. The MUST having the ratio of the diameter of the circular tablet to the thickness thereof in a range of 1 : 0.7 to 1 : 1.3 in accordance with the present invention can completely fill the internal space of the hard capsule without forming any void, and thus, a larger amount of a pharmaceutical composition can be charged even in a smaller capsule.

In addition, when the ratio of the cylinder height to the total thickness of the MUST is in a specific range, the tableting characteristics of the MUST improve, and hence can improve the speed of the tableting process.

Specifically, the cylinder height is equal to the total length of the tablet minus the length of both hemispherical ends (top and bottom) combined, as shown in Fig. 2. If the ratio of the cylinder height in the total thickness is too high, the tablet would form a rectangular shape which can deteriorate the flowability of the tablet, and thereby causing a trouble during the packing process. If the ratio of the cylinder height in the total thickness is too low, the tablet would be prone to shatter during the tableting process. Therefore, the ratio of the thickness to the cylinder height of the MUST in accordance with the present invention is in a range of 1 : 0.3 to 1 : 0.9, preferably 1 : 0.5 to 1 : 0.8.

Further, the MUST is a mini tablet having a small size, and thus the internal space of the capsule comprises at least 4 or more of mini tablets, preferably 4 to 40 MUSTs per each pharmaceutically active ingredient.

Accordingly, the diameter of the MUST in the composite formulation according to the present invention is less than the internal diameter of the hard capsule body, and preferably less than or equal to 1/2 of the internal diameter of the hard capsule.

If the diameter of the MUST is too large (e.g., greater than 1/2 of the internal diameter of the hard capsule body), it becomes difficult to charge the tablets in the internal space of the capsule, and also the total number of tablets to be charged in the internal space of the capsule decreases, thereby inhibiting an improvement in dissolution rate or a reduction in standard deviation of dissolution rates. Even after the tablets were charged, it does not allow the formation of desired packing arrangement, and thus cause voids to form within the capsule. On the other hand, when the diameter of the MUST is too small (e.g., less than 1 mm), the amount charged in a single tablet is limited, and also the physical properties of the tablet may be largely affected by the environmental variables during the tableting process.

Therefore, the MUST in accordance with the present invention has a diameter in a range of 1 mm to 4 mm, preferably in a range of 1.5 mm to 3 mm. If the diameter of the tablet is in the said range, the desirable packing arrangement can be achieved, which allows the maximization of the contents to be charged in a capsule, and it also yields an improvement in dissolution rate due to having a plurality of tablets.

The composite formulation of the present invention has a good charge rate as compared to conventional hard capsules which are charged with granule or pellets.

The charge rate of a capsule can be calculated by the weight of material charged in the capsule over the volume of the capsule body. For example, when 150 mg of a composition is charged in a No. 2 capsule (vol.: 0.37 mL), the charge rate is approximately 0.41 g/mL. Generally, it is difficult to achieve a charge rate of 0.6 g/mL or greater if granule or pellet material is charged due to the low density of the material charged or the voids existing in the pellet. In contrast, the composite formulation according to the present invention has a charge rate of 0.6 g/mL to 1.0 g/mL, and thus having a charge rate of 0.6 g/mL or greater, which make it possible to reduce the size of a hard capsule, rendering it readily administered to patients.

Further, the composite formulation in accordance with the present invention has a significantly less porosity (the rate of empty space) as compared to a conventional capsule which is charged with typical tablets having a diameter of 5 mm or greater. Since there is an inverse relationship between the charge rate and the porosity value, the MUST of the present invention can be charged in the internal space of the capsule at an optimum level.

In accordance with one aspect of the present invention, there is provided a hard capsule composite formulation comprising a plurality of MUSTs whose diameter is less than or equal to 1/2 of the internal diameter of the hard capsule body, and is in a range of 1 mm to 4 mm. In such formulations, the diameter of the MUST may be in a range of 1.5 mm to 3 mm.

According to another aspect of the present invention, there is provided a hard capsule composite formulation comprising a plurality of MUST whose ratio of its thickness to the cylinder height is in a range of 1 : 0.7 to 1 : 1.3, and the diameter thereof is in a range of 1 mm to 4 mm. In such formulations, the ratio of the thickness to the cylinder height of the MUST may be in a range of 1 : 0.8 to 1 : 1.2, and the diameter thereof may be in the range 1.5 mm to 3 mm.

According to still another aspect of the present invention, there is provided a hard capsule composite formulation comprising a plurality of MUST whose diameter is less than or equal to 1/2 of the internal diameter of the hard capsule body, and the ratio of the thickness of MUST to the cylinder height thereof may be in a range of 1 : 0.8 to 1 : 1.2.

Any two or more drugs known in the art can be employed as active ingredient for the MUSTs of the present invention. The drug used in the present invention may be selected from the same or different drug groups. Examples of the drug employable include antipyretics agents, analgesics, anti-inflammatory agents and muscle relaxants such as tramadol, naproxen, ibuprofen, dexibuprofen, aspirin, acetaminophen, indomethacin, diclofenac sodium, aceclofenac, ketoprofen, isopropyl antipyrine, phenacetin, flubiprofen, phenylbutazone, etodolac, celecoxib, etoricoxib, eperisone, and pharmaceutically acceptable salts thereof; antiulcer agents such as omeprazole, esomeprazole, pantoprazole, cimetidine, famotidine, ranitidine, nizatidine, roxatidine, and pharmaceutically acceptable salts thereof; cardiovascular agent or vasodilators such as losartan, ibesartan, candesartan, telmisartan, valsartan, nifedipine, amlodipine, verapamil, captopril, diltiazem hydrochloride, propranolol, oxprenolol, nitroglycerin, enalapril, and pharmaceutically acceptable salts thereof; antidiabetic agents such as metformin, glimepiride, sitagliptin, rosiglitazone, pioglitazone, and pharmaceutically acceptable salts thereof; anti-hyperlipidemic agents such as simvastatin, rosuvastatin, atorvastatin, and pharmaceutically acceptable salts thereof; antibiotics such as ampicillin, amoxicillin, cephalexin, cefuroxime, cefdinir, cefadroxil, cefprozil, cefpodoxime, cefditoren, cefaclor, cefixime, cefradine, loracarbef, ceftibuten, cefatrizine, cefcapene, erythromycins, tetracyclines, quinolones, and pharmaceutically acceptable salts thereof; antitussives or anti-asthmatic agents such as montelukast, theophylline, aminophylline, codeine phosphate, methylephedrine hydrochloride, dextromethorpan, noscapine, salbutamol, ambroxol, levodropropizine, clenbuterol, tebutalin, and pharmaceutically acceptable salts thereof; antiemetic agents or GIT regulators such as ondansetron, metoclopramide, domperidone, trimebutine maleate, cisapride, levosulpiride, and pharmaceutically acceptable salts thereof; anti-impotence agents such as sildenafil, vardenafil, tadalafil, udenafil, and pharmaceutically acceptable salts thereof; and anti-dementia agents such as donepezil, galantamine, rivastigmine, acetyl carnitine, memantine, xaliproden, and pharmaceutically acceptable salts thereof.

Besides, anti-BPH agents such as tamsulosin; antimigraine agents such as zolmitriptan and rizatriptan; psychostimulants; antimicrobials; antihistamines such as cetirizine, levocetirizine, and loratadine; antidiabetic agents; antiallergic agents; contraceptives; vitamin supplements; anticoagulants such as clopidogrel; muscle relaxants; brain metabolism enhancers; diuretics such as torsemide, and furosemide; antiepileptic agents such as gabapentin, pregabalin, valproate, topiramate, carbamazepine, lamotrigine, oxcarbazepine; and antiparkinson drugs such as selegiline; antipsychotics such as risperidone, ziprasidone, quetiapine, olanzapine, clozapine, and paliperidone, and pharmaceutically acceptable salts thereof may be employed in the present invention. Also, biological agents such as oral vaccines may be employed in the present invention.

Preferably, the active ingredient may be selected from the group consisting of levocetirizine, montelukast, ambroxol, levodropropizine, losartan, ibersartan, amlodipine, rosuvastatin, atorvastatin, aspirin, clopidogrel, aceclofenac, eperison, esomeprazole, naproxen, and pharmaceutically acceptable salts thereof.

In the inventive composite formulation, the MUST may further comprise pharmaceutically acceptable additives selected from the group consisting of a pharmaceutically acceptable diluent, disintegrating agent, binder, stabilizer, lubricant, coloring agent, and a mixture thereof.

The diluent may be selected from the group consisting of microcrystalline cellulose, lactose, Ludipress®, mannitol, monocalcium phosphate, starch, low-substituted hydroxypropyl cellulose, and a mixture thereof. The amount of diluent employed may be about 1 to 99 wt%, preferably about 5 to 90 wt%, based on the total weight of the tablet.

The disintegrating agent may be any material that safely swells in a liquid environment, which is selected from the group consisting of crospovidone, sodium starch glycolate, croscarmellose sodium, low-substituted hydroxypropyl cellulose, starch, alginate, or its sodium salt, or a mixture thereof. In a preferred embodiment of the present invention, the disintegrating agent is selected from the group consisting of low-substituted hydroxypropyl cellulose, crospovidone, sodium starch glycolate, croscarmellose sodium, and a mixture thereof. The amount of disintegrating agent employed may be about 1 to 30 wt%, preferably about 2 to 15 wt%, based on the total weight of the tablet.

The binder may be selected from the group consisting of hydroxypropyl cellulose, hydroxypropyl methylcellulose, polyvinyl pyrrolidone, copovidone, macrogol, light anhydrous silicic acid, synthetic aluminum silicate, silicate derivatives such as calcium silicate or magnesium metasilicate aluminate, phosphates such as calcium hydrogen phosphate, carbonates such as calcium carbonate, and a mixture thereof, and the amount of binder employable may be about 1 to 30 wt%, preferably about 2 to 15 wt%, based on the total weight of the tablet.

The stabilizer may be an antioxidant, acidifying agent, or basifying agent.

Specific examples of the antioxidant include butylated hydroxytoluene (BHT), butylated hydroxyanisole (BHA), ascorbic acid, ascorbyl palmitate, ethylenediaminetetraacetic acid (EDTA), sodium pyrosulfite, and a mixture thereof; particularly butylated hydroxytoluene is preferred. Specific examples of the acidifying agent include organic acids such as fumaric acid, citric acid, tartaric acid, succinic acid, lactic acid, malic acid, tosylic acid, oxalic acid, ascorbic acid, glutamic acid, alginic acid, maleic acid and adipic acid; inorganic acids such as hydrochloric acid, sulfuric acid, nitric acid, phosphoric acid, acetic acid and boric acid, and a mixture thereof, preferably fumaric acid, citric acid, tartaric acid, and phosphoric acid. Examples of the basifying agent include arginine, lysine, histidine, meglumine, aluminum magnesium silicate, aluminum magnesium metasilicate, or basic minerals such as NaHCO₃, CaCO₃, MgCO₃, KH₂PO₄, K₂HPO₃, and tribasic calcium phosphate, preferably NaHCO₃, CaCO₃, MgCO₃ or a mixture thereof.

The stabilizer can be selected depending on the nature of the pharmaceutically active ingredients, and the amount of stabilizing agent employed may be 0.01 to 10 wt%, based on the total amount of the selected pharmaceutically active ingredients.

The lubricant may be selected from the group consisting of stearic acid, metal stearates such as calcium stearate and magnesium stearate, talc, colloidal silica, sucrose esters of fatty acids, hydrogenated vegetable oil, high melting point wax, glyceryl fatty acid esters, glycerol dibehenate and a mixture thereof, and the amount of lubricant employed may be in a range of about 0.02 to 5 wt%, preferably about 0.3 to 3 wt%, based on the total weight of the tablet.

The coloring agent may be selected from the group consisting of red iron oxide pigments, yellow iron oxide pigments, titanium dioxide, Blue No. 1, Blue No. 2, and a mixture thereof, and the amount of coloring agent employed may be in a range of about 0.001 to 2 wt%, preferably about 0.01 to 1.5 wt%, based on the total weight of the tablet.

The inventive composite formulation comprising a plurality of MUSTs, and thus have a very fast dissolution rate without experiencing a reduction in initial dissolution. Generally, a conventional hard capsule formulation requires the disintegration time for the capsule, hence there is a drawback of an increase in the deviation of dissolution test results due to the slowdown of the initial dissolution rate (within 15 min). Thus, it may be difficult to expect the same bioequivalence of a single dosage form from a composite formulation if it comprises a drug which requires fast absorption rate, e.g., maximum drug concentration time (Tₘₐₓ) of 1 to 2 hrs. However, the inventive composite formulation comprising a plurality of tablets having a small size which can quickly disintegrate simultaneously, and thus there is no delay in initial dissolution rate.

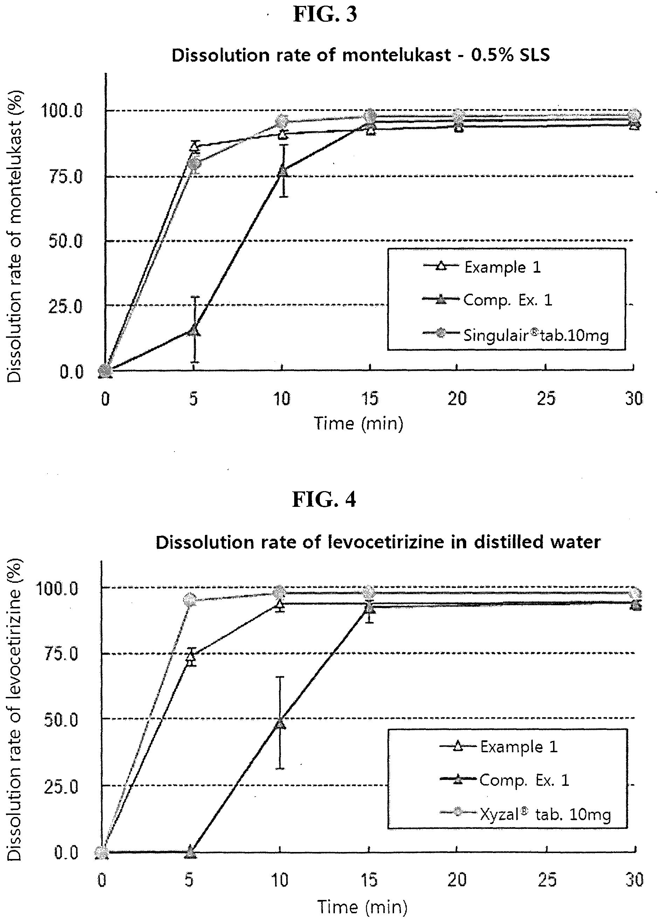

Accordingly, one or more active ingredients of the inventive composite formulation are immediate release drugs, one or more active ingredients having *in vitro* initial dissolution rate of 30% or more within 5 min of administration, and *in vitro* initial dissolution rate of 80% or more within 10 min of administration (*see* Figs. 3 to 5).

Further, the inventive composite formulation completely separates each active ingredient, securing improved dissolution rate and a good stability upon long-term storage. This advantageous effect can be enhanced further by coating the MUST. Accordingly, the MUST of the present invention may be coated with a polymer film coating layer so as to physically prevent any possible interaction between two or more active ingredients.

Any conventional polymer that can form a film coating may be used in the film coating layer of the present invention. Specific examples include water soluble polymers such as polyvinyl alcohol, hydroxyethyl cellulose, hypromellose, polyvinylpyrrolidone, and a mixture thereof; water insoluble polymers such as hypromellose phthalate (HPMCP), polyvinyl acetate (e.g., Kollicoat® SR 30D), water insoluble polymethacrylate copolymer [such as, poly(ethyl acrylate-methyl methacrylate) copolymer (e.g., Eudragit® NE30D), poly(ethyl acrylate-methyl methacrylate-trimethylaminoethyl methacrylate chloride) copolymer (e.g., Eudragit® RSPO], ethyl cellulose, cellulose ester, cellulose ether, cellulose acylate, cellulose diacylate, cellulose triacylate, cellulose acetate, cellulose diacetate, cellulose triacetate, and a mixture thereof.

The employment of such polymer not only serves to separate active ingredients from one another, but also serves to allow forming MUST with different dissolution rates (e.g., immediate/sustained or immediate/enteric) in the same capsule. In this case, the MUST is coated with said polymer, and then can be charged in a desirable ratio as the drug designer had intended so as to regulate the dissolution rates between the same drug groups or between different drug groups. This advantageous effect was made possible by employing a plurality of mini tablets as MUST of the in the present invention.

The amount of polymer may be adjusted in order to provide a tablet having an appropriate size and dissolution rate in an efficient manner, which is preferably about 1 to 50 wt%, more preferably about 1 to 20 wt %, based on the total weight of the tablet. Each tablet is completely separated and forms an independent dosage form, preventing any interaction between the tablets. Also, in the analysis of the stability of active ingredients prepared in accordance with the present invention, it would be sufficient to analyze the stability of each tablet contained in a capsule by a conventional method for analyzing a single medicine, instead of any special method therefor.

Also, the present invention provides a method for preparing the hard capsule composite formulation, which comprises the steps of: (1) preparing a MUST comprising a pharmaceutically active ingredient; and (2) encapsulating a plurality of the MUSTs in the hard capsule such that the hard capsule composite formulation comprises two or more pharmaceutically active ingredients. The method may further comprise an additional step of coating the MUST with a polymer film during the step (1) of the method above.

Hereinafter, the present invention is described more specifically by the following examples.

### Example 1: Preparation of Composite Formulation I

**- Levocetirizine layer -**

| | |
|---|---|
| Levocetirizine dihydrochloride | 5.0 mg |
| Ludipress® | 60.5 mg |
| Microcrystalline cellulose | 8.1 mg |
| Citric acid | 3.0 mg |
| Croscarmellose sodium | 5.0 mg |
| Light anhydrous silicic acid | 0.5 mg |
| Magnesium stearate | 0.9 mg |
| Opadry® Y-1-7000 | 2.0 mg |
| Distilled water | (10.0 mg) |

**- Montelukast layer -**

| | |
|---|---|
| Montelukast sodium | 10.4 mg (montelukast, 10 mg) |
| D-mannitol | 45.4 mg |
| Microcrystalline cellulose | 92.0 mg |
| Light anhydrous silicic acid | 2.4 mg |
| Hydroxypropyl cellulose | 4.0 mg |
| Sodium starch glycolate | 8.4 mg |
| Magnesium Stearate | 3.4 mg |
| Hypromellose | 1.5 mg |
| Hydroxypropyl cellulose | 1.5 mg |
| Titanium dioxide | 0.96 mg |
| Red iron oxide | 0.004 mg |
| Yellow iron oxide | 0.036 mg |
| Distilled water | (40.0 mg) |

The levocetirizine-containing tablet layer was prepared as described below. Levocetirizine dihydrochloride, Ludipress® (BASF), microcrystalline cellulose, citric acid, croscarmellose sodium, light anhydrous silicic acid, and magnesium stearate were sieved and admixed, and then the resulting mixture was pressed into a tablet using a tablet press machine with the die diameter of 2.0 mm, to yield 10 MUSTs, wherein each tablet has the weight of 8.3 mg, the thickness of about 2.0 mm, and the cylinder height of 1.3 mm.

Separately, a coating solution was prepared by dissolving Opadry® Y-1-7000 in distilled water, and the coating solution was applied on the levocetirizine-containing MUSTs prepared above. The total weight of 10 MUSTs thus obtained was 85 mg, and the total weight of levocetirizine therein was 5 mg.

Meanwhile, the montelukast-containing tablet layer was prepared as described below. Montelukast sodium, D-mannitol, microcrystalline cellulose, light anhydrous silicic acid, hydroxypropyl cellulose, sodium starch glycolate, and magnesium stearate were sieved and admixed, and then the resulting mixture was pressed into a tablet using a tablet press machine with the die diameter of 2.0 mm, to yield 20 MUSTs, wherein each tablet has the weight of 8.3 mg, the thickness of about 2.0 mm, and the cylinder height of 1.3 mm.

Separately, a coating solution was prepared by dissolving hypromellose, hydroxypropyl cellulose, titanium dioxide, red iron oxide, and yellow iron oxide in distilled water, and the coating solution was applied on the montelukast-containing MUSTs prepared above. The total weight of 20 MUSTs thus obtained was 170 mg, and the total weight of montelukast therein was 10 mg.

The two different MUSTs prepared above, 10 levocetirizine-containing MUSTs and 20 montelukast-containing MUSTs, were charged in the capsule body of a No. 1 hard capsule primarily made up with gelatin to produce a hard capsule formulation comprising 10 mg of montelukast and 5 mg of levocetirizine.

### Example 2: Preparation of Composite Formulation II

**- Ambroxol layer -**

| | |
|---|---|
| Ambroxol hydrochloride | 30.0 mg |
| Lactose hydrate | 22.7 mg |
| Pregelatinized starch | 22.7 mg |
| Povidone K-30 | 1.4 mg |
| Distilled water | (20.0 mg) |
| Light anhydrous silicic acid | 0.4 mg |
| Magnesium stearate | 0.8 mg |

**- Levodropropizine layer -**

| | |
|---|---|
| Levodropropizine | 60.0 mg |
| Lactose hydrate | 46.6 mg |
| Microcrystalline cellulose | 47.0 mg |
| Sodium starch glycolate | 5.6 mg |
| Magnesium stearate | 0.8 mg |

The ambroxol-containing tablet layer was prepared as described below. Ambroxol hydrochloride, lactose hydrate, and pregelatinized starch were admixed, added with a binding solution prepared by dissolving povidone K-30 in distilled water, and the mixture was wet granulated. Light anhydrous silicic acid and magnesium stearate were added thereto, and the mixture was pressed into a tablet using a tablet press machine with the die diameter of 2.0 mm, to yield 10 MUSTs, wherein each tablet has the weight of 7.8 mg and the thickness of about 2.0 mm, and the cylinder height of 1.3 mm. The total weight of 10 MUSTs thus obtained was 78 mg, and the total weight of ambroxol hydrochloride therein was 30 mg.

Meanwhile, the levodropropizine-containing tablet layer was prepared as described below. Levodropropizine, lactose hydrate, microcrystalline cellulose, sodium starch glycolate, and magnesium stearate were sieved and admixed, and then the resulting mixture was pressed into a tablet using a tablet press machine with the die diameter of 2.0 mm, to yield 20 MUSTs, wherein each tablet has the weight of 8.0 mg, the thickness of about 2.0 mm, and the cylinder height of 1.4 mm. The total weight of 20 MUSTs thus obtained was 160 mg, and the total weight of levodropropizine therein was 60 mg.

The two different MUSTs prepared above, 10 ambroxol hydrochloride-containing MUSTs and 20 levodropropizine-containing MUSTs, were charged in the capsule body of a No. 1 hard capsule primarily made up with gelatin to produce a hard capsule formulation comprising 30 mg of ambroxol hydrochloride and 60 mg of levodropropizine.

### Example 3: Preparation of Composite Formulation III

**- Losartan layer -**

| | |
|---|---|
| Losartan potassium | 50.0 mg |
| Ludipress® | 41.5 mg |
| Copovidone | 3.7 mg |
| Light anhydrous silicic acid | 1.0 mg |
| Croscarmellose sodium | 3.0 mg |
| Magnesium stearate | 0.8 mg |
| Opadry® Y-1-7000 | 2.0 mg |
| Distilled water | (10.0 mg) |

**- Amlodipine layer -**

| | |
|---|---|
| Amlodipine camsylate | 15.68 mg (amlodipine 10 mg) |
| Mannitol | 40.0 mg |
| Microcrystalline cellulose | 36.92 mg |
| Sodium starch glycolate | 2.4 mg |
| Hydroxypropyl cellulose | 3.0 mg |
| Magnesium stearate | 2.0 mg |
| Opadry® Y-1-7000 | 2.0 mg |
| Distilled water | (10.0 mg) |

The losartan-containing tablet layer was prepared as described below. Losartan potassium, Ludipress® (BASF), copovidone, croscarmellose sodium, light anhydrous silicic acid, and magnesium stearate were sieved and admixed, and then the mixture was pressed into a tablet using a tablet press machine with the die diameter of 2.0 mm, to yield 12 MUSTs, wherein each tablet has the weight of about 8.3 mg and the thickness of about 2.0 mm, and the cylinder height of 1.2 mm.

Separately, a coating solution was prepared by dissolving Opadry® Y-1-7000 in distilled water, and the coating solution was applied on the losartan-containing MUSTs prepared above. The total weight of 12 MUSTs thus obtained was 102 mg, and the total weight of losartan therein 50 mg.

Meanwhile, the amlodipine-containing tablet layer was prepared as described below. Amlodipine camsylate, mannitol, microcrystalline cellulose, sodium starch glycolate, hydroxypropyl cellulose, and magnesium stearate were sieved and admixed, and then the resulting mixture was pressed into a tablet using a tablet press machine with the die diameter of 2.0 mm, to yield 12 MUSTs, wherein each tablet has the weight of about 8.3 mg and the thickness of about 2.0 mm, and the cylinder height of 1.3 mm.

Separately, a coating solution was prepared by dissolving Opadry® Y-1-7000 in distilled water, and the coating solution was applied on the amlodipine-containing MUSTs prepared above. The total weight of 12 MUSTs thus obtained was 102 mg, and the total weight of amlodipine therein was 10 mg.

The two different MUSTs prepared above, 12 losartan-containing MUSTs and 12 amlodipine-containing MUSTs, were charged in the capsule body of a No. 2 hard capsule primarily made up with gelatin to produce a hard capsule formulation comprising 50 mg of losartan and 10 mg of amlodipine.

### Example 4: Preparation of Composite Formulation IV

**- Rosuvastatin layer -**

| | |
|---|---|
| Rosuvastatin calcium | 10.4 mg (rosuvastatin 10 mg) |
| Lactose hydrate | 44.7 mg |
| Microcrystalline cellulose | 22.8 mg |
| Crospovidone | 4.3 mg |
| Magnesium stearate | 0.8 mg |
| Opadry® Y-1-7000 | 3.0 mg |
| Red iron oxide | 0.1 mg |
| Distilled water | (15.0 mg) |

**- Aspirin layer -**

| | |
|---|---|
| Aspirin | 100.0 mg |
| Microcrystalline cellulose | 26.0 mg |
| Pregelatinized starch | 13.0 mg |
| Light anhydrous silicic acid | 1.5 mg |
| Stearic acid | 0.5 mg |
| Hypromellose phthalate | 17.0 mg |
| Titanium dioxide | 1.7 mg |
| Acetylated monoglyceride | 0.3 mg |
| Ethanol | (90.0 mg) |
| Distilled water | (180.0 mg) |

The rosuvastatin-containing tablet layer was prepared as described below. Rosuvastatin calcium, lactose hydrate, microcrystalline cellulose, crospovidone, and magnesium stearate were sieved and admixed, and then the mixture was pressed into a tablet using a tablet press machine with the die diameter of 2.0 mm, to yield 10 MUSTs, wherein each tablet has the weight of about 8.3 mg, the thickness of about 2.0 mm, and the cylinder height of 1.3 mm.

Separately, a coating solution was prepared by dissolving Opadry® Y-1-7000 in distilled water, and the coating solution was applied on the rosuvastatin-containing MUSTs prepared above. The total weight of 10 MUSTs thus obtained was 86 mg, and the total weight of rosuvastatin therein was 10 mg.

Meanwhile, the aspirin-containing tablet layer was prepared as described below. Aspirin, microcrystalline cellulose, pregelatinized starch, and light anhydrous silicic acid were admixed. Stearic acid was added as a lubricant to the resulting mixture, and then the mixture was pressed into a tablet using a tablet press machine with the die diameter of 2.0 mm, to yield 20 MUSTs, wherein each tablet has the weight of about 7.05 mg, the thickness of about 2.0 mm, and the cylinder height of 1.2 mm.

Separately, a coating solution was prepared by dissolving hypromellose phthalate, titanium dioxide, and acetylated monoglyceride in a mixed solvent of ethanol and acetone, and the coating solution was applied on the aspirin-containing MUSTs prepared above (enteric coating). The total weight of 20 MUSTs thus obtained was 160 mg, and the total weight of aspirin therein was 100 mg.

The two different MUSTs prepared above, 10 rosuvastatin-containing MUSTs and 20 aspirin-containing MUSTs, were charged in the capsule body of a No. 1 hard capsule primarily made up with gelatin to produce a hard capsule formulation comprising 10 mg of rosuvastatin and 100 mg of aspirin.

### Example 5: Preparation of Composite Formulation V

**- Clopidogrel layer -**

| | |
|---|---|
| Clopidogrel hydrogen sulfate | 97.9 mg (clopidogrel 75 mg) |
| D-mannitol | 40.0 mg |
| Low-substituted hydroxypropyl cellulose | 17.1 mg |
| Sucrose esters of fatty acids | 5.0 mg |
| Opadry® 32K-14834 | 4.0 mg |
| Distilled water | (15.0 mg) |

**- Aspirin layer -**

| | |
|---|---|
| Aspirin | 100.0 mg |
| Microcrystalline cellulose | 26.0 mg |
| Pregelatinized starch | 13.0 mg |
| Light anhydrous silicic acid | 1.5 mg |
| Stearic acid | 0.5 mg |
| Hypromellose phthalate | 17.0 mg |
| Titanium dioxide | 1.7 mg |
| Acetylated monoglyceride | 0.3 mg |
| Ethanol | (90.0 mg) |
| Distilled water | (180.0 mg) |

The clopidogrel-containing tablet layer was prepared as described below. Clopidogrel hydrogen sulfate, D-mannitol, low-substituted hydroxypropyl cellulose, and sucrose esters of fatty acids were sieved and admixed, and then the mixture was pressed into a tablet using a tablet press machine with the die diameter of 2.0 mm, to yield 20 MUSTs, wherein each tablet has the weight of about 8.0 mg, the thickness of about 2.0 mm, and the cylinder height of 1.3 mm.

Separately, a coating solution was prepared by dissolving Opadry® 32K-14834 in distilled water, and the coating solution was applied on the clopidogrel-containing MUSTs prepared above. The total weight of 20 MUSTs thus obtained was 164 mg, and the total weight of clopidogrel therein was 75 mg.

Meanwhile, the aspirin-containing layer was prepared as described below. Aspirin, microcrystalline cellulose, pregelatinized starch, and light anhydrous silicic acid were admixed. Stearic acid was added as a lubricant to the resulting mixture, and then the mixture was pressed into a tablet using a tablet press machine with the die diameter of 2.0 mm, to yield 20 MUSTs, wherein each tablet has the weight of about 7.05 mg, the thickness of about 2.0 mm, and the cylinder height of 1.2 mm.

Separately, a coating solution was prepared by dissolving hypromellose phthalate, titanium dioxide, and acetylated monoglyceride in a mixed solvent of ethanol and acetone, and the coating solution was applied on the aspirin-containing MUSTs prepared above (enteric coating). The total weight of 20 MUSTs thus obtained was 160 mg, and the total weight of aspirin therein was 100 mg.

The two different MUSTs prepared above, 20 clopidogrel-containing MUSTs and 20 aspirin-containing MUSTs, were charged in the capsule body of an elongated No. 1 hard capsule primarily made up with gelatin to produce a hard capsule formulation comprising 75 mg of clopidogrel and 100 mg of aspirin.

### Comparative Example 1: Preparation of Composite Formulation VI

**- Levocetirizine layer -**

| | |
|---|---|
| Levocetirizine dihydrochloride | 5.0 mg |
| Ludipress® | 60.5 mg |
| Microcrystalline cellulose | 8.1 mg |
| Citric acid | 3.0 mg |
| Croscarmellose sodium | 5.0 mg |
| Light anhydrous silicic acid | 0.5 mg |
| Magnesium stearate | 0.9 mg |
| Opadry® Y-1-7000 | 2.0 mg |
| Distilled water | (10.0 mg) |

**- Montelukast layer -**

| | |
|---|---|
| Montelukast sodium | 10.4 mg (montelukast 10 mg) |
| D-mannitol | 45.4 mg |
| Microcrystalline cellulose | 92.0 mg |
| Light anhydrous silicic acid | 2.4 mg |
| Hydroxypropyl cellulose | 4.0 mg |
| Sodium starch glycolate | 8.4 mg |
| Magnesium Stearate | 3.4 mg |
| Hypromellose | 1.5 mg |
| Hydroxypropyl cellulose | 1.5 mg |
| Titanium dioxide | 0.96 mg |
| Red iron oxide | 0.004 mg |
| Yellow iron oxide | 0.036 mg |
| Distilled water | (40.0 mg) |

The levocetirizine-containing tablet layer was prepared as described below. Levocetirizine dihydrochloride, Ludipress®, microcrystalline cellulose, citric acid, croscarmellose sodium, light anhydrous silicic acid, and magnesium stearate were sieved and admixed, and then the mixture was pressed into a tablet using a tablet press machine with the die diameter of 5.0 mm, to yield a tablet. Then, a coating solution prepared by dissolving Opadry® Y-1-7000 in distilled water was applied on the tablet to produce the levocetirizine tablet. The total weight of the tablet thus obtained was 85 mg, and the total weight of levocetirizine therein was 5 mg.

Meanwhile, the montelukast-containing tablet layer was prepared as described below. Montelukast sodium, D-mannitol, microcrystalline cellulose, light anhydrous silicic acid, hydroxypropyl cellulose, sodium starch glycolate, and magnesium stearate were sieved and admixed, and then the resulting mixture was pressed into a tablet using a tablet press machine with the dye diameter of 5.0 mm, to yield two tablets. Separately, a coating solution was prepared by dissolving hypromellose, hydroxypropyl cellulose, titanium dioxide, red iron oxide, yellow iron oxide in distilled water, and the coating solution was applied on the tablet to produce the montelukast tablet. The total weight of the tablet thus obtained was 170 mg, and the total weight of montelukast therein was 10 mg.

The two different tablets prepared above, 1 levocetirizine tablet and 2 montelukast tablets, were charged in the capsule body of a No. 1 hard capsule primarily made up with gelatin to produce a hard capsule formulation comprising 5 mg of levocetirizine and 10 mg of montelukast.

### Comparative Example 2: Preparation of Composite Formulation VII

**- Ambroxol layer -**

| | |
|---|---|
| Ambroxol hydrochloride | 30.0 mg |
| Lactose hydrate | 22.7 mg |
| Pregelatinized starch | 22.7 mg |
| Povidone K-30 | 1.4 mg |
| Distilled water | (20.0 mg) |
| Light anhydrous silicic acid | 0.4 mg |
| Magnesium stearate | 0.8 mg |

**- Levodropropizine layer -**

| | |
|---|---|
| Levodropropizine | 60.0 mg |
| Lactose hydrate | 46.6 mg |
| Microcrystalline cellulose | 47.0 mg |
| Sodium starch glycolate | 5.6 mg |
| Magnesium stearate | 0.8 mg |

The ambroxol-containing tablet layer was prepared as described below. Ambroxol hydrochloride, lactose hydrate, and pregelatinized starch were admixed, added with a binding solution prepared by dissolving povidone K-30 in distilled water, and then the mixture was wet granulated. Light anhydrous silicic acid and magnesium stearate were added thereto, and the mixture was pressed into a tablet using a tablet press machine with the die diameter of 5.0 mm, to yield a tablet. The total weight of the tablet thus obtained was 78 mg, and the total weight of ambroxol hydrochloride therein was 30 mg.

Meanwhile, the levodropropizine-containing tablet layer was prepared as described below. Levodropropizine, lactose hydrate, microcrystalline cellulose, sodium starch glycolate, and magnesium stearate were sieved and admixed, and then the resulting mixture was pressed into a tablet using a tablet press machine with the die diameter of 5.0 mm, to yield two tablets. The total weight of the tablets thus obtained was 160 mg, and the total weight of levodropropizine therein was 60 mg.

The two different tablets prepared above, 1 ambroxol tablet and 2 levodropropizine tablets, were charged in the capsule body of a No. 1 hard capsule primarily made up with gelatin to produce a hard capsule formulation comprising 30 mg of ambroxol hydrochloride and 60 mg of levodropropizine.

### Test Example 1: Dissolution Test of Montelukast and Levocetirizine

The composite formulations comprising montelukast and levocetirizine prepared in Example 1 and Comparative Example 1, and Singulair® tablet (MSD, 10 mg) and Xyzal® tablet (Korea UCB Co., 5 mg) as reference drugs for montelukast and levocetirizine, respectively, were subjected to drug dissolution test using six test vessels for each drug under the following conditions. The results are shown in Table 1, and Figs. 3 and 4.

### <Test Conditions>

- Dissolution medium:
   for montelukast = 0.5% sodium lauryl sulfate (SLS) solution, 900 mL
   for levocetirizine = distilled water, 900 mL
- Dissolution-test system: paddle, 75 rpm
- Temperature: 37°C

### <Analytical Conditions- Simultaneous determination of montelukast and levocetirizine >

- Column: stainless steel column filled with 5 µm octadecylsilyl silica gel for liquid chromatography (Inertsil C8, 4.6 x 150 mm, 5 µm)
- Mobile phase: 0.025 M potassium dihydrogen phosphate (pH 6.6), acetonitrile (40 : 60, v/v)
- Detector : ultraviolet spectrophotometer (225 nm)
- Flow rate: 1.0 mL/min
- Injection volume: 10 µL
- Column temperature: 45 °C

**[Table 1]**

| Component | Time | Example 1 | Comp. Ex. 1 | Singulair® tab. | Xyzal® tab. |
|---|---|---|---|---|---|
| Montelukast | 5 min | 86.3 ± 2.4% | 13.2 ± 12.6% | 79.5 ± 3.4% | - |
| | 10 min | 90.9 ± 1.3% | 77.7 ± 9.8% | 95.4 ± 2.4% | - |
| | 15 min | 92.3 ± 1.1% | 95.2 ± 2.4% | 97.6 ± 1.7% | - |
| Levocetirizine | 5 min | 74.2 ± 3.4% | 0.2 ± 0.0% | - | 95.3 ± 1.5% |
| | 10 min | 94.1 ± 2.9% | 48.9 ± 17.4% | - | 98.0 ± 2.1 % |
| | 15 min | 94.0 ± 1.3% | 92.6 ± 5.6% | - | 97.8 ± 0.9% |

As shown in Table 1 and Figs. 3 and 4, the composite formulation prepared in Example 1 comprising MUST exhibited much higher initial dissolution rates (within 10 min) than that of Comparative Example 1 comprising conventional tablets, which was even similar to that of reference drugs, Singulair® tablet (montelukast) and Xyzal® tablet (levocetirizine).

In addition, the dissolution test results of montelukast and levocetirizine of Example 1 demonstrated significantly less standard deviation values than the result of dissolution test of Comparative Example 1, and thus smaller changes in body absorption rates can be expected from the inventive composite formulation. Therefore, it can also be expected that it would be easier for the inventive hard capsule composite formulation comprising MUST to be bioequivalent with their reference drugs Singulair® and Xyzal®.

### Test Example 2: Dissolution Test of Ambroxol

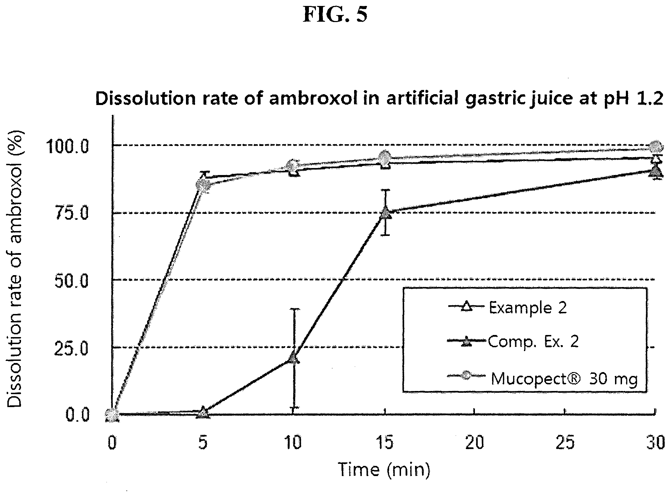

The composite formulations comprising ambroxol and levodropropizine prepared in Example 2 and Comparative Example 2, and Mucopect® tablet (Boehringer Ingelheim, 30 mg) as a reference drug for ambroxol were subjected to drug dissolution test using six test vessels for each drug under the following conditions. The results are shown in Table 2, and Fig 5.

### <Test Condition>

- Dissolution medium: pH 1.2 artificial gastric juice, 900 mL
- Dissolution-test system: paddle, 50 rpm
- Temperature: 37 °C

### <Analytical Conditions- ambroxol>

- Column: stainless steel column filled with octadecylsilanized silica gel for 5 µm liquid chromatography (Waters ODS-2, 4.6 mm x 50 mm, 5 µm)
- Mobile phase: 0.05 M potassium dihydrogen phosphate (adjusted to pH 3.0 using phosphoric acid), methanol (88 : 12, v/v)
- Detector: ultraviolet spectrophotometer (254 nm)
- Flow rate: 1.0 mL/min
- Injection amount: 10 µL
- Column temperature: 30 °C

**[Table 2]**

| Component | Time | Example 2 | Comp. Ex. 2 | Mucopect® tab. |
|---|---|---|---|---|
| Ambroxol | 5 min | 88.4 ± 2.3% | 1.2 ± 0.1% | 85.3 ± 2.6% |
| | 10 min | 90.6 ± 1.9% | 21.4 ± 18.4% | 92.4 ± 2.1% |
| | 15 min | 93.4 ± 0.8% | 75.4 ± 8.3% | 95.4 ± 1.8% |

As shown in Table 2 and Fig. 5, the composite formulation prepared in Example 2 comprising MUST exhibited much higher initial dissolution rates (within 10 min) and much smaller changes in the standard deviation values than that of Comparative Example 2 which is charged with a conventional tablet.

Therefore, it can be expected that if ambroxol, whose initial dissolution rate is considered critical to its given Tₘₐₓ of 0.25 to 1 hr, is prepared in the form of the inventive hard capsule composite formulation comprising MUSTs, then it would be easier for the inventive composite formulation to be bioequivalent with its reference drug, Mucopect® tablet, and smaller changes in body absorption rates is also expected.

### Test Example 3: Absorption Test for Montelukast and Levocetirizine

The composite formulations comprising montelukast and levocetirizine prepared in Example 1, and Singulair® tablet 10 mg and Xyzal® tablet 5 mg as reference drugs for montelukast and levocetirizine, respectively, were orally administered to test animals for bioavailability test under the following conditions.

The test animals were healthy, male, 20-month-old Beagle dogs with a body weight of 12 ± 2 kg and 5 dogs were alloted per test group. The dogs were placed in a cage, allowed to have free access to commercial dog feed (400 g per day), and then fasted over a period of 14 hrs prior to the experiment. The dogs were divided into two groups: Group 1 (Example 1) and Group 2 (Singulair® tab.+ Xyzal® tab.). Each dogs were orally administered with the corresponding formulation, and forcefully administered with 40 mg of water. Blood samples (2 mL) were taken from cephalic vein using a tube with an anticoagulant (1,000 IU/mL, heparin 5µl) at 0 (initial), 0.25, 0.5, 1, 2, 3, 4, 8, 10, 24 and 48 hrs after the oral administration. All blood samples were centrifuged (12,000 rpm, 2 min, Eppendorf) to plasma, and contained in a freezer at -20 °C for later analysis of each sample by LC-MS under the following conditions:
Column: Halo C18 (2.1 × 50 mm, 2.7 µm)
Mobile phase: methanol, 10 mM ammonium formate (85 : 15, v/v)
Injection amount: 10 µL
Detection: Turbo Ion spray Ionization mode (positive)

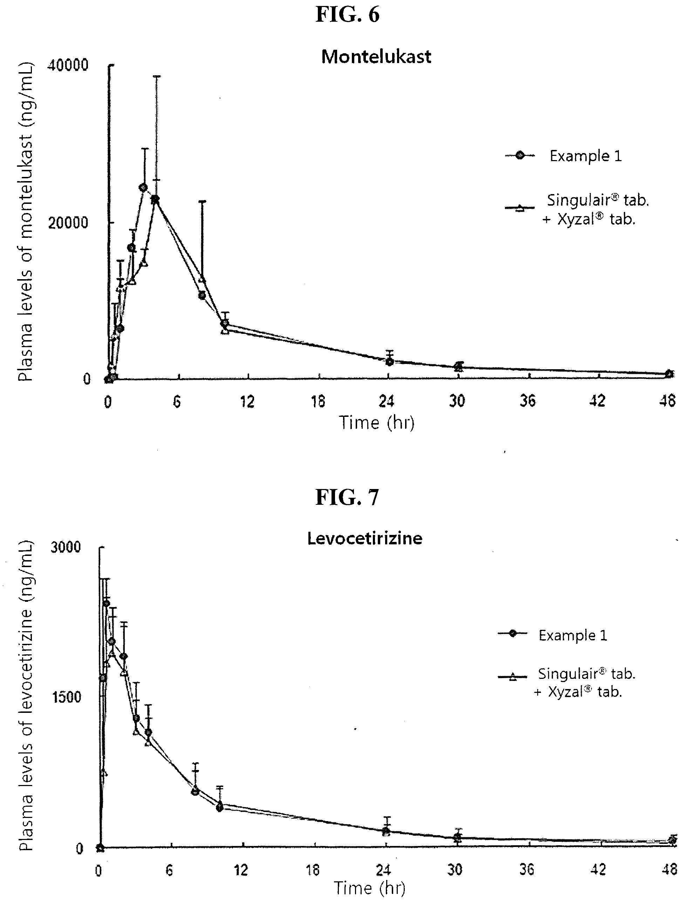

Cₘₐₓ and Tₘₐₓ were obtained from the plasma concentration versus time curve, and AUC from 0 to 24 hrs after the administration was calculated according to trapezoidal rule using the curve. The results are shown in Table 3, and Figs. 6 and 7.

**[Table 3]**

| | | AUC^{*1} | Cₘₐₓ ^{*2} | Tₘₐₓ^{*3} |
|---|---|---|---|---|
| Montelukast | Example 1 | 231319 ± 35054 | 24833 ± 4366 | 3.3 ± 0.6 |
| | Singulair® + Xyzal® | 228783 ± 80531 | 25200 ± 13612 | 3.5 ± 0.6 |
| Lev ocetirizine | Example 1 | 17106 ± 6340 | 2452 ± 259 | 0.5 ± 0.1 |
| | Singulair® + Xyzal® | 16200 ± 2582 | 2290 ± 472 | 0.8 ± 0.3 |
| *1 : Area under the curve (initial to 24 hr) | | | | |
| *2 : Peak plasma concentration | | | | |
| *3 : Time to peak plasma concentration | | | | |

As shown in Table 3, and Figs. 6 and 7, the composite formulation of Example 1 had an equivalent or superior bioavailability than taking the two single formulation reference tablets simultaneously.

The composite formulation in accordance with Example 1 resulted similar AUC and Cₘₐₓ values of montelukast and levocetirizine to its reference drugs, Singulair® tablet and Xyzal® tablet, and showed no delay in Tₘₐₓ values, but rather, slightly Tₘₐₓ value.

## Claims

1. A hard capsule composite formulation comprising two or more pharmaceutically active ingredients, wherein each pharmaceutically active ingredient is contained in a multi-unit spheroidal tablet (MUST) having a diameter in a range of 1 to 4 mm and 4 to 40 MUSTs per each pharmaceutically active ingredient are encapsulated in the hard capsule; and wherein each MUST has a diameter to thickness ratio in a range of 1:0.7 to 1:1.3 and a thickness to cylinder height ratio in a range of 1:0.3 to 1:0.9.

2. The hard capsule composite formulation of claim 1, wherein the diameter of each MUST is less than or equal to 1/2 of the internal diameter of the hard capsule.

3. The hard capsule composite formulation of claim 1, wherein the thickness to cylinder height ratio is in a range of 1:0.5 to 1:0.8.

4. The hard capsule composite formulation of claim 1, wherein the hard capsule is hard capsule No. 0, hard capsule No. 1, hard capsule No. 2, hard capsule No. 3, or hard capsule No. 4.

5. The hard capsule composite formulation of claim 1, wherein each pharmaceutically active ingredient is selected from the group consisting of levocetirizine, montelukast, ambroxol, levodropropizine, losartan, ibersartan, amlodipine, rosuvastatin, atorvastatin, aspirin, clopidogrel, aceclofenac, eperison, esomeprazole, naproxen, and pharmaceutically acceptable salts thereof.

6. The hard capsule composite formulation of claim 1, wherein each MUST comprises a pharmaceutically acceptable additive.

7. The hard capsule composite formulation of claim 6, wherein the pharmaceutically acceptable additive is selected from the group consisting of a pharmaceutically acceptable diluents, disintegrating agent, binder, stabilizer, lubricant, coloring agent, and a mixture thereof.

8. The hard capsule composite formulation of claim 1, wherein each MUST is coated with a polymer film coating layer.

9. The hard capsule composite formulation of claim 1, wherein one or more of the pharmaceutically active ingredients are immediately released.

10. A method for preparing the hard capsule composite formulation of claim 1, which comprises:
(1) preparing a MUST having a diameter in a range of 1 to 4 mm which comprises a pharmaceutically active ingredient; and
(2) encapsulating 4 to 40 MUSTs in the hard capsule such that the hard capsule composite formulation comprises two or more pharmaceutically active ingredients, wherein each MUST has a diameter to thickness ratio in a range of 1:0.7 to 1:1.3 and a thickness to cylinder height ratio in a range of 1:0.3 to 1:0.9.

11. The method of claim 10, which further comprises coating each MUST with a polymer film during step (1).

## Patentansprüche

1. Hartkapsel mit zusammengesetzter Formulierung, umfassend zwei oder mehr pharmazeutisch aktive Bestandteile, wobei jeder pharmazeutisch aktive Bestandteil in einer sphäroidalen Tablette mit mehrfachen Einheiten (multi-unit spheroidal tablet, MUST) enthalten ist, die einen Durchmesser in einem Bereich von 1 bis 4 aufweist, und 4 bis 40 MUST pro jeden pharmazeutisch aktiven Bestandteil in der Hartkapsel eingekapselt sind; und wobei jede MUST ein Verhältnis von Durchmesser zu Dicke im Bereich von 1 : 0,7 bis 1 : 1,3 sowie ein Verhältnis von Dicke zu Zylinderhöhe in einem Bereich von 1 : 0,3 bis 1 : 0,9 aufweist.

2. Hartkapsel mit zusammengesetzter Formulierung nach Anspruch 1, wobei der Durchmesser jeder MUST kleiner oder gleich 1/2 des Innendurchmessers der Hartkapsel ist.

3. Hartkapsel mit zusammengesetzter Formulierung nach Anspruch 1, wobei das Verhältnis der Dicke zu der Zylinderhöhe im Bereich von 1 : 0,5 bis 1 : 0,8 liegt.

4. Hartkapsel mit zusammengesetzter Formulierung nach Anspruch 1, wobei die Hartkapsel Hartkapsel Nr. 0, Hartkapsel Nr. 1, Hartkapsel Nr. 2, Hartkapsel Nr. 3 oder Hartkapsel Nr. 4 ist.

5. Hartkapsel mit zusammengesetzter Formulierung nach Anspruch 1, wobei jeder pharmazeutisch aktive Bestandteil ausgewählt ist aus der Gruppe bestehend aus Levocetirizin, Montelukast, Ambroxol, Levodropropizin, Losartan, Ibersartan, Amlodipin, Rosuvastatin, Atorvastatin, Aspirin, Clopidogrel, Aceclofenac, Eperison, Esomeprazol, Naproxen und pharmazeutisch verträglichen Salzen davon.

6. Hartkapsel mit zusammengesetzter Formulierung nach Anspruch 1, wobei jede MUST ein pharmazeutisch verträgliches Additiv davon umfasst.

7. Hartkapsel mit zusammengesetzter Formulierung nach Anspruch 6, wobei das pharmazeutisch verträgliche Additiv ausgewählt ist aus der Gruppe bestehend aus pharmazeutisch verträglichen Verdünnungsmitteln, Zerfallsmitteln, Bindemitteln, Stabilisiermitteln, Gleitmitteln, färbenden Mitteln und einer Mischung davon.

8. Hartkapsel mit zusammengesetzter Formulierung nach Anspruch 1, wobei jede MUST mit einer Überzugsschicht eines Polymerfilms beschichtet ist.

9. Hartkapsel mit zusammengesetzter Formulierung nach Anspruch 1, wobei ein oder mehrere der pharmazeutisch aktiven Bestandteile sofort freigesetzt werden.

10. Verfahren zum Herstellen einer Hartkapsel mit zusammengesetzter Formulierung nach Anspruch 1, wobei das Verfahren umfasst:
(1) Herstellen einer MUST mit einem Durchmesser im Bereich von 1 bis 4 mm, die einen pharmazeutisch aktiven Bestandteil umfasst; und
(2) Einkapseln von 4 bis 40 MUST in die Hartkapsel derart, dass die Hartkapsel mit zusammengesetzter Formulierung zwei oder mehrere pharmazeutisch aktive Stoffe umfasst, wobei jede MUST ein Verhältnis von Durchmesser zu Dicke im Bereich von 1 : 0,7 bis 1 : 1,3 sowie ein Verhältnis von Dicke zu Zylinderhöhe in einem Bereich von 1 : 0,3 bis 1 : 0,9 aufweist.

11. Verfahren nach Anspruch 10, das ferner Beschichten jeder MUST mit einem Polymerfilm während des Schritts (1) umfasst.

## Revendications

1. Formulation composite à capsule dure comprenant deux ingrédients pharmaceutiquement actifs ou plus, dans laquelle chaque ingrédient pharmaceutiquement actif est contenu dans un comprimé sphéroïdal à plusieurs unités (MUST) ayant un diamètre dans un intervalle de 1 à 4 mm et 4 à 40 MUSTs par chaque ingrédient pharmaceutiquement actif sont encapsulés dans la capsule dure; et dans laquelle chaque MUST a un rapport de diamètre sur épaisseur dans un intervalle de 1:0,7 à 1:1,3 et un rapport d'épaisseur sur hauteur de cylindre dans un intervalle de 1:0,3 à 1:0,9.

2. Formulation composite à capsule dure selon la revendication 1, dans laquelle le diamètre de chaque MUST est inférieur ou égal à 1/2 du diamètre interne de la capsule dure.

3. Formulation composite à capsule dure selon la revendication 1, dans laquelle le rapport d'épaisseur sur hauteur de cylindre est dans un intervalle de 1:0,5 à 1:0,8.

4. Formulation composite à capsule dure selon la revendication 1, dans laquelle la capsule dure est une capsule dure No. 0, une capsule dure No. 1, une capsule dure No. 2, une capsule dure No. 3 ou une capsule dure No. 4.

5. Formulation composite à capsule dure selon la revendication 1, dans laquelle chaque ingrédient pharmaceutiquement actif est choisi dans le groupe constitué de: lévocétirizine, montélukast, ambroxol, lévodropropizine, losartan, irbésartan, amlodipine, rosuvastatine, atorvastatine, aspirine, clopidogrel, acéclofénac, épérisone, ésoméprazole, naproxène et des sels pharmaceutiquement acceptables de ceux-ci.

6. Formulation composite à capsule dure selon la revendication 1, dans laquelle chaque MUST comprend un additif pharmaceutiquement acceptable.

7. Formulation composite à capsule dure selon la revendication 6, dans laquelle l'additif pharmaceutiquement acceptable est choisi dans le groupe constitué d'un diluant pharmaceutiquement acceptable, d'un agent de désintégration, d'un liant, d'un stabilisant, d'un lubrifiant, d'un agent colorant et d'un mélange de ceux-ci.

8. Formulation composite à capsule dure selon la revendication 1, dans laquelle chaque MUST est enrobé avec une couche d'enrobage de film de polymère.

9. Formulation composite à capsule dure selon la revendication 1, dans laquelle un ou plusieurs des ingrédients pharmaceutiquement actifs sont immédiatement libérés.

10. Méthode pour la préparation de la formulation composite à capsule dure selon la revendication 1, qui comprend:
(1) la préparation d'un MUST ayant un diamètre dans un intervalle de 1 à 4 mm qui comprend un ingrédient pharmaceutiquement actif; et
(2) l'encapsulation de 4 à 40 MUSTs dans la capsule dure de sorte que la formulation composite à capsule dure comprend deux ingrédients pharmaceutiquement actifs ou plus, dans laquelle chaque MUST a un rapport de diamètre sur épaisseur dans un intervalle de 1:0,7 à 1:1,3 et un rapport d'épaisseur sur hauteur de cylindre dans un intervalle de 1:0,3 à 1:0,9.

11. Méthode selon la revendication 10, qui comprend en outre l'enrobage de chaque MUST avec un film de polymère pendant l'étape (1).
